# EUROPEAN PATENT APPLICATION

(11) **EP 2 692 386 A1**
(43) Date of publication of application: **05.02.2014**
(21) Application number: 11862642.3
(22) Date of filing: 29.11.2011
(51) Int. Cl.: A61M 25/00

(54) **DEVICE FOR OTORHINOLARYNGOLOGICAL THERAPY**

(30) Priority: 29.03.2011 JP 2011072998
(71) Applicant: Terumo Kabushiki Kaisha, Tokyo 151-0072 (JP)
(72) Inventor: SHIMIZU, Katsuhiko, Ashigarakami-gun Kanagawa 259-0151 (JP); KINOSHITA, Yasushi, Ashigarakami-gun Kanagawa 259-0151 (JP); SUEHARA, Satoru, Ashigarakami-gun Kanagawa 259-0151 (JP); OOTANI, Yousuke, Tokyo 100-0005 (JP); ARASTOO, Riyaheh, Ashigarakami-gun Kanagawa 259-0151 (JP); KUMOYAMA, Kenichi, Ashigarakami-gun Kanagawa 259-0151 (JP)
(74) Representative: Dossmann, Gérard
(86) International application number: PCT/JP2011/077583
(87) International publication number: WO 2012/132114

(57) **Abstract**

[Subject] There is provided an otorhinolaryngological device which enables easy positioning of the expansion body inside the nasal cavity, without need for radioscopy.

[Solution] An otorhinolaryngological treatment device includes: a flexible elongated body 11 to be introduced into a paranasal sinus through a nasal cavity; an expansion body 12 which is provided on the elongated body 11 and has an effective expansive section 12a capable of radial expansive deformation within a natural ostium between the nasal cavity and the paranasal sinus to thereby force open a stenosed part of the natural ostium; and imaging unit 13 for obtaining an image on the front side of a distal end of the elongated body 11, which is provided integrally with the elongated body 11 on the distal side of the elongated body 11 relative to the expansion body 12.

## Description

### DEVICE AND METHOD

### Technical Field

The present invention relates to an otorhinolaryngological treatment device to be used for treatment of sinusitis or the like and an otorhinolaryngological treatment method thereof.

### Background Art

A paranasal sinus is an intraosseous cavity adjacent to a nasal cavity, and communicates with the nasal cavity through a small hole called natural ostium. Secretions, bacteria and the like in the paranasal sinus are excreted through the natural ostium. When the mucous membrane in the nasal cavity is swollen due to common cold-induced rhinitis or allergic rhinitis or the like or the inside of the nasal cavity is narrowed due to deflected nasal septum or hypertrophic rhinitis or the like, however, the natural ostium is stenosed and chronic inflammation is generated in the paranasal sinus. Such a disease is called sinusitis. Conventionally, the method for treatment of sinusitis has generally been a surgical operation in which the lesion causing stenosis of the natural ostium is removed by use of forceps, a drill or the like while confirming the video image of the inside of the nasal cavity through an endoscope. In recent years, however, a sinusitis treatment method based on the use of a balloon catheter and not including a surgical operation has been developed, and has been drawing attention from the viewpoint of minimal invasiveness to the patient.

In the treatment method developed recently, a guide wire and a balloon catheter are sequentially inserted into the nasal cavity, and, after it is radioscopically confirmed that the balloon catheter has been disposed in the natural ostium, the balloon catheter is expanded to force open the stenosed part of the natural ostium. According to this treatment method, the communicating passage between the nasal cavity and the paranasal sinus can be recovered while greatly alleviating the bleeding in the nasal cavity, damage to the mucous membrane, etc. In connection with this technique, a following patent literature 1 proposes a balloon catheter in which a plurality of radiopaque markers for marking the balloon proximal end, distal end and the like are disposed on the inner surface of the balloon. On the other hand, from the viewpoint of prevention of exposure of the patient to X-rays, there is an increasing demand for a balloon catheter which enables easy positioning (alignment) of the balloon inside the nasal cavity without relying on radioscopy.

### Citation List

### Patent Literature

Patent Literature1: Japanese Unexamined Patent Application Publication No.2008-513125

### Summary of Invention

### Technical Problem

The present invention has been made aiming at solving the above-mentioned problems involved in the related art. Accordingly, it is an object of the present invention to provide an otorhinolaryngological treatment method in which positioning of an expansion body inside a nasal cavity can be easily carried out without need for radioscopy.

### Solution to Problem

The purpose of describing the above present invention is achieved by any one of the following means.
(1) An otorhinolaryngological treatment device including: a flexible elongated body to be inserted into a living body; an expansion body which is provided on the elongated body and has an effective expansive section capable of radial expansive deformation within a natural ostium located between a nasal cavity and a paranasal sinus in the living body to force open a stenosed part of the natural ostium; and imaging unit for obtaining an image on the front side of a distal end of the elongated body, which is provided integrally with the elongated body on the distal side of the elongated body relative to the expansion body.
(2) The treatment device according to (1), includes a long-shaped holding member contained in the elongated body and having a rigidity by which the external shape of the elongated body in the living body is held such that at least a part of the elongated body is bent into a direction crossing an axial direction of the elongated body.
(3) The treatment device according to (2), the imaging unit includes an image sensor part disposed inside the elongated body, and a protective member which has an opening part for leading out a connection cable electrically connected to the image sensor part and which covers the image sensor part; and a distal end of the protective member is disposed on the distal side of the elongated body relative to a part of the connection cable which is led out through the opening part.
(4) The treatment device according to (2) or (3), the holding member is disposed at least at a position in the extending direction of the elongated body where the effective expansive section of the expansion body is located.
(5) The treatment device according to any one of (1) to (4), the elongated body is provided with a marker for confirming that the elongated body is moved along the extending direction of the natural ostium by the distance between an image obtaining plane of the imaging unit and the effective expansive section.
(6) The treatment device according to (5), the marker may be configured to be movable along the extending direction of the elongated body and to be fixable to the elongated body.
(7) The treatment device according to (5) or (6), the marker is configured to be detachable from the elongated body.
(8) The treatment device according to any one of (5) to (7), the marker may have graduations for indicating the distance.
(9) The treatment device according to any one of (1) to (8), the elongated body may be provided with a first lighting unit for illuminating the front side of the distal end of the elongated body.
(10) The treatment device according to any one of (1) to (9), the expansion body has a light-permeability; and a second light for illuminating the outer periphery of the expansion body through the expansion body is disposed inside the expansion body.
(11) The treatment device according to any one of (1) to (10), a center position in the radial direction of the expansion body and a center position of an image obtaining plane of the imaging unit are coaxially-arranged with each other.
(12) The treatment device according to any one of (1) to (11), the imaging unit may be located at the distal end of the elongated body.
(13) A method of dilating a stenosed part of a natural ostium located between a nasal cavity and a paranasal sinus in a living body, including: a step of introducing into the living body a elongated body provided with an expansion body capable of expansive deformation and contractive deformation and imaging unit for obtaining an image of the inside of the living body, disposed on the distal side of the elongated body relative to the expansion body, each of which is integrated with the elongated body; a guiding step of obtaining an image on the front side of a distal end of the elongated body by the imaging unit, and guiding an effective expansive section of the expansion body for applying a pressure to the stenosed part of the natural ostium, into the stenosed part of the natural ostium on the basis of the obtained image; and a dilating step of expanding the expansion body to thereby dilate the stenosed part of the natural ostium.
(14) The method of dilating a stenosed part according to (12), the guiding step includes a step of positioning an image obtaining plane of the imaging unit on the proximal side of an entrance to the paranasal sinus, and thereafter advancing the elongated body distally by the distance between the image obtaining plane and the effective expansive section of the expansion body thereby to position the effective expansive section with respect to the stenosed part of the natural ostium.
(15) The method of dilating a stenosed part according to (13) or (14), the dilating step includes at least a step of causing expansive deformation and contractive deformation in the expansion body while advancing the elongated body distally or retreating the elongated body proximally.
(16) The method of dilating a stenosed part according to any one of (13) to (15), may further include a suctioning step for suctioning a fluid in the living body through a lumen disposed in the elongated body in a state where the elongated body has been introduced into the living body.

### Effects of Invention

In the treatment device according to (1), by advancing the elongated body within the nasal cavity while observing the image obtained by the imaging unit, it is possible to easily confirm the arrival of the elongated body at the paranasal sinus, so that the expansion body provided on the elongated body can be positioned accurately in the stenosed part of the natural ostium. According to the treatment device, therefore, the stenosed part of the natural ostium can be forced open by the expansion body more assuredly, and a therapeutic effect on sinusitis can be enhanced. Further, by the treatment device, the elongated body can be prevented from being excessively advanced into the paranasal sinus, so that safety in treating sinusitis can be enhanced. In addition, the treatment device does not need any radioscopic device, and therefore enables treatment of sinusitis even in a small hospital or the like where such a special device is not installed.

In the treatment device according to (2), the elongated body can be reshaped at the time of introduction into a living body. Further, the external shape (reshaped shape) of the elongated body can be maintained in the living body. Accordingly, the elongated body and the expansion body can be easily and assuredly guided into the vicinity of a stenosed part of the natural ostium, irrespectively of differences in introduction path to each paranasal sinus to be treated and individual differences in anatomical structure from patient to patient.

In the treatment device according to (3), where the distal end of the holding member is disposed on the distal side of the elongated body relative to a part of the connection cable which is led out through the opening part in the protective member, a starting point of reshaping into a curved shape or the like by means of the holding member can be prevented from being formed in the led-out part of the connection cable from the opening part. This ensures that the connection cable can be prevented from suffering a damage such as breaking of wire. In addition, since reshaping into a curved shape or the like does not occur at the elongated body portion where the protective member is disposed, the image sensor part contained in the protective member can be prevented from suffering a breakage or a deterioration in function.

In the treatment device according to (4), where the holding member is disposed at least at a position in the extending direction of the elongated body where the effective expansive section of the expansion body is located, the outer peripheral surface of the expansion body can be reshaped in conjunction with the elongated body. Therefore, the treatment device becomes applicable not only to a natural ostium having a rectilinear inner cavity shape but also to a natural ostium having a curved inner cavity shape. Accordingly, while using a single treatment device, it is possible to widely cope with natural ostia which are located at different parts of a living body.

In the treatment device according to (5), where the elongated body is provided with the marker as above-mentioned, after the arrival of the image obtaining plane of the imaging unit at the paranasal sinus is confirmed through the image obtained by the imaging unit, the operator can further advance the elongated body by the distance between the image obtaining plane and the effective expansive section of the expansion body while observing the marker provided on the proximal operating section, whereby the effective expansive section can be disposed over the whole range of the stenosed part. Therefore, the stenosed part of the natural ostium can be forced open more assuredly, and the therapeutic effect on sinusitis can be more enhanced.

In the treatment device according to (6), where the marker is movable as above-mentioned, the position of the marker can be varied according to the naris (opening of nasal cavity), so that positioning of the expansion body can be carried out more accurately. Therefore, the stenosed part of the natural ostium can be forced open more reliably, so that the therapeutic effect on sinusitis can be enhanced further.

In the treatment device according to (7), where the marker is detachable from the elongated body, it is possible to remove the marker before or after the positioning of the expansion body. This promises better usability of the treatment device.

In the treatment device according to (8), where the marker has graduations for indicating the distance, the elongated body can be more accurately advanced by the distance. Therefore, the stenosed part of the natural ostium can be forced open more assuredly, so that the therapeutic effect on sinusitis can be enhanced further.

In the treatment device according to (9), where the elongated body is provided with the first lighting unit as above-mentioned, a favorable visual field for the imaging unit can be secured by the light emitted from the first lighting unit, so that the expansion body provided on the elongated body can be more accurately disposed in the stenosed part of the natural ostium. Therefore, the therapeutic effect on sinusitis and safety in treatment can be further enhanced.

In the treatment device according to (10), where the expansion body is light-transmitting and the second light as above-mentioned is provided inside the expansion body, the light emitted from the second lighting unit enters the paranasal sinus at the time when the expansion body arrives at the paranasal sinus through the stenosed part of the natural ostium. Therefore, the elongated body can be advanced while checking whether or not the light from the second lighting unit has entered the paranasal sinus, whereby the expansion body can be disposed over the whole range of the stenosed part. Accordingly, the stenosed part can be forced open more assuredly, so that the therapeutic effect on sinusitis can be more enhanced.

In the treatment device according to (11), where the expansion body and the image obtaining plane of the imaging unit are coaxially-arranged, the image obtaining plane is always located at the center of the path of the elongated body, so that penetrability of the elongated body into the nasal cavity can be enhanced. Besides, since a favorable visual field can be secured, the arrival of the elongated body at the paranasal sinus can be confirmed more accurately. Therefore, the therapeutic effect on sinusitis and safety in treatment can be enhanced further.

In the treatment device according to (12), where the imaging unit is provided at the distal end of the elongated body, the image obtaining plane is always located in the forefront of the path of the elongated body, the arrival of the elongated body at the paranasal sinus can be confirmed more accurately. Therefore, the therapeutic effect on sinusitis and safety in treatment can be enhanced further.

In the method of dilating a stenosed part according to (13), ensures that the elongated body can be prevented from being excessively advanced into the paranasal sinus, so that safety in treatment of paranasal sinus can be enhanced. In addition, the method does not need any radioscopic device, and therefore enables treatment of sinusitis even in a small hospital or the like where such special device is not installed.

In the method of dilating a stenosed part according to (14), where the elongated body is advanced further by the distance between the image obtaining plane of the imaging unit and the effective expansive section of the expansion body after confirmation of the arrival of the image obtaining plane at the paranasal sinus through the image obtained by the imaging unit, as above-mentioned, the effective expansive section can be disposed over the whole range of the stenosed part. This ensures that the stenosed part of the natural ostium can be forced open more assuredly, so that the therapeutic effect on sinusitis can be more enhanced.

In the method of dilating a stenosed part according to (15), where expansive deformation and contractive deformation are caused in the expansion body while the elongated body being advanced or retreated, the stenosed part of the natural ostium can be forced open more securely.

In the method of dilating a stenosed part according to (16), where the fluid in the living body is suctioned through the lumen disposed in the elongated body in the condition where the elongated body has been introduced into the living body, as above-mentioned, adhesion of body fluid or secretions onto the image obtaining plane of the imaging unit can be prevented, so that a clearer image can be obtained.

### Brief Description of The drawings

[FIG. 1] FIG. 1 is a schematic view of a treatment system according to a first embodiment of the present invention.
[FIG. 2] FIG. 2 is a side view of a treatment device (catheter) according to the first embodiment of the present invention.
[FIG. 3] FIG. 3 is a side view of the treatment device (catheter) according to the first embodiment of the present invention.
[FIG. 4] FIG. 4 is a longitudinal sectional view of a major part of the catheter of FIG. 2.
[FIG. 5] FIG. 5 is a cross-sectional view along line V-V of FIG. 4.
[FIG. 6] FIG. 6 is a longitudinal sectional view showing a modification of the treatment device according to the first embodiment of the present invention.
[FIG. 7] FIG. 7 is schematic views illustrating a method of positioning the treatment device according to the first embodiment of the present invention.
[FIG. 8] FIG. 8 is a longitudinal sectional view of a major part of a treatment device according to a second embodiment of the present invention.
[FIG. 9] FIG. 9 is schematic views illustrating a method of positioning the treatment device according to the second embodiment of the present invention.
[FIG. 10] FIG. 10 is a schematic view showing an embodiment of the method of treating sinusitis by use of the treatment device according to the first embodiment of the present invention.
[FIG. 11] FIG. 11 is schematic views illustrating an embodiment of the method of treating sinusitis by use of the treatment device according to the first embodiment of the present invention.
[FIG. 12] FIG. 12 is a schematic view showing an embodiment of the method of treating sinusitis by use of the treatment device according to the first embodiment of the present invention.
[FIG. 13] FIG. 13 is a schematic view showing an embodiment of the method of treating sinusitis by use of the treatment device according to the first embodiment of the present invention.
[FIG. 14] FIG. 14 is schematic views illustrating an embodiment of the method of treating sinusitis by use of the treatment device according to the second embodiment of the present invention.
[FIG. 15] FIG. 15 is a schematic view showing another aspect of the method of treating sinusitis by use of a guide catheter together with the treatment device according to the embodiment of the present invention.
[FIG. 16] FIG. 16 is a schematic view showing yet another aspect of the method of treating sinusitis by use of the guide catheter together with the treatment device according to the embodiment of the present invention.
[FIG. 17] FIG. 17 is a schematic vies showing yet another aspect of the method of treating sinusitis by use of a guide wire together with the treatment device according to the embodiment of the present invention.
[FIG. 18] FIG. 18 is a schematic view showing yet another aspect of the method of treating sinusitis by use of a guide wire together with the treatment device according to the embodiment of the present invention.
[FIG. 19] FIG. 19 is a side view of a treatment device (catheter) according to a third embodiment of the present invention.
[FIG. 20] FIG. 20 is a longitudinal sectional view of a major part of the treatment device (catheter) according to the third embodiment of the present invention.
[FIG. 21] FIGS 21A and 21B are enlarged sectional views of the treatment device according to the third embodiment of the present invention, wherein FIG. 21A is a sectional view along line VI-VI of FIG. 20, and FIG. 21B along line VII-VII.

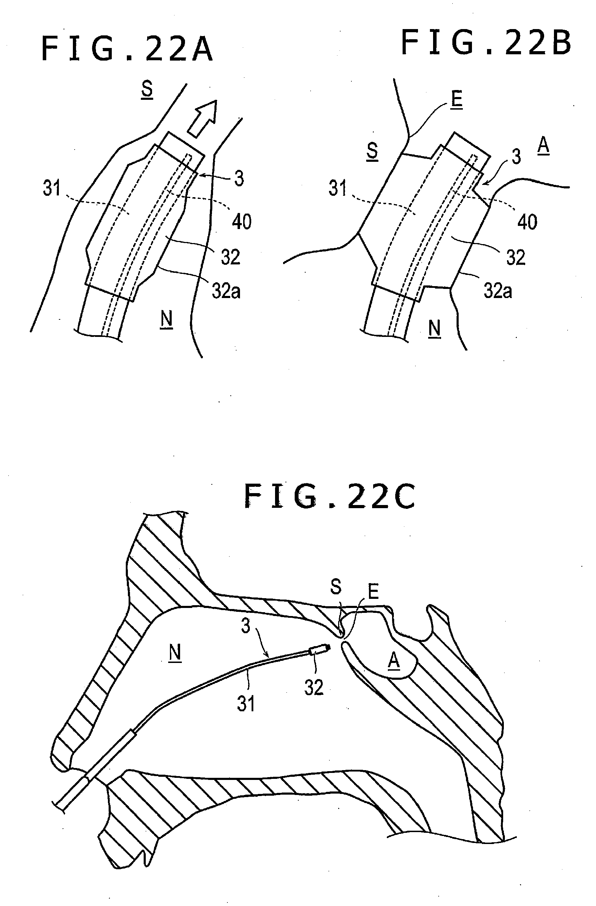

[FIG. 22] FIGS. 22A to 22C are schematic views illustrating an embodiment of the method of treating sinusitis by use of the treatment device according to the third embodiment of the present invention, wherein FIG. 22A is a partial enlarged view showing a condition where an expansion body of the treatment device is introduced in the vicinity of a natural ostium, FIG. 22B is a partial enlarged view showing a condition where a stenosed part of the natural ostium is dilated by the expansion device possessed by the treatment device, and FIG. 22C is a views showing schematically a condition where the treatment device is introduced into an external nose;
[FIG. 23] FIGS. 23A and 23B are enlarged sectional views for illustrating Modification (1) of the treatment device according to the third embodiment of the present invention.
[FIG. 24] FIG. 24 is a longitudinal sectional view of a major part of Modification (2) of the treatment device according to the third embodiment of the present invention.

### Description of Embodiments

Now, embodiments of the present invention will be described below referring to the drawings. For convenience of description, ratios of the dimensions of components in individual drawings and ratios of the dimensions of the same component in the plurality of drawings are varied, as required, and are therefore not necessarily conforming to the actual ratios.

### <STRUCTURE OF TREATMENT DEVICE ACCORDING TO THE PRESENT INVENTION>

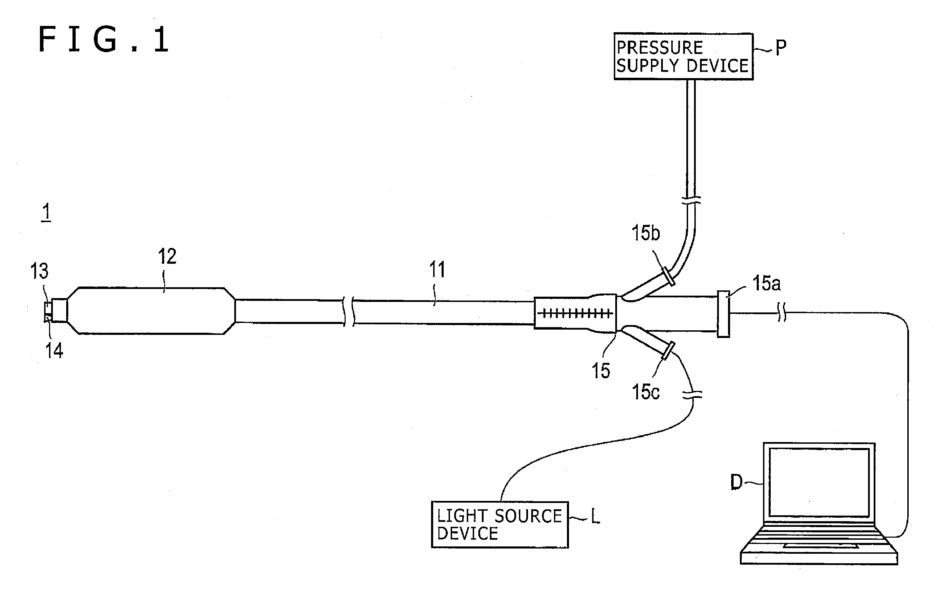

FIG. 1 is a schematic illustration of the general structure of a treatment system including a catheter 1 as a treatment device according to a first embodiment of the present invention. As shown in FIG. 1, the catheter 1 includes a first elongated body 11 constituting a main body thereof, a balloon 12 as an expansion body for forcing open a stenosed part of a natural ostium, a CCD camera 13 as imaging unit for obtaining an image of the inside of a nasal cavity, an LED light 14 as a lighting unit for illuminating the inside of the nasal cavity, and a hub 15 having the function of a proximal operating section to be operated by the operator as well as the function as a connection port for connection to an external apparatus.

Here, the term "CCD camera" means a digital video camera using a CCD image sensor as an imaging element. The hub 15 includes an image port 15a as the connection port for connection to the external apparatus, a pressure supply port 15b, and a light supply port 15c. These ports will be described later. Incidentally, the catheter 1 is inserted into the patient's nasal cavity from its end portion where the CCD camera 13 is disposed, to be used for treatment of sinusitis. In the following description, the end portion of the catheter 1 for insertion into the nasal cavity will be referred to as distal end, and the end portion on the opposite side will be referred to as proximal end.

As shown in FIG. 1, the catheter 1 is connected to a display device D such as an LCD through the image port 15a of the hub 15, to a pressure supply device P such as an indeflator through the pressure supply port 15b, and to a light source device L through the light supply port. Here, the display device D displays thereon an image obtained by the CCD camera 13. The pressure supply device P supplies the balloon 12 with a liquid or the like. The light source device L supplies the LED light 14 with electric power.

Incidentally, the imaging unit in the present embodiment is not restricted to the CCD camera, but may be any of a digital video camera using other imaging element such as a CMOS image sensor, an image fiber for obtaining and transmitting images by means of optical fibers, and an imaging system for transmitting images by means of an objective lens and an optical system including a plurality of relay lenses. The "image obtaining plane" in the cases of using various cameras or optical imaging systems means a predetermined part of the imaging unit disposed so as confront an organ in a living body at the time of introduction into the inside of the living body, and the image obtaining plane may be a distal-end surface of a protective member of the image sensing element or a lens, for example.

The lighting unit in the present embodiment is not limited to the LED light, but may be other lighting units such as a halogen lamp and a high-intensity discharge lamp (HID lamp). Apart from the example shown in FIG. 1 where the LED lamp 14 is attached to the distal end of the catheter 1, the catheter 1 can also be configured such that light generated by the light source device L is guided to its distal end through a light guide made by glass or plastic.

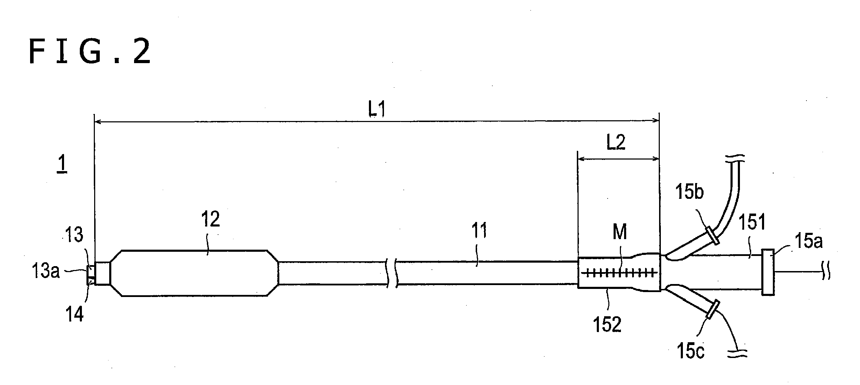

FIG. 2 is a side view of the catheter 1 shown in FIG. 1. As shown in FIG. 2, the balloon 12 is disposed in the vicinity of the distal end of the first elongated body 11, and the CCD camera 13 and the LED light 14 are attached to the first elongated body 11 at a position on the distal side relative to the balloon 12. The CCD camera 13 has an image obtaining plane 13a perpendicular to the extending direction of the catheter 1, and can obtain an image on the front side (distal side) of the distal end of the catheter 1. Besides, for securing a visual field of the CCD camera 13, the LED light 14 is configured to illuminate the front side (distal side) of the image obtaining plane 13a of the CCD camera 13. Incidentally, the CCD camera 13 and the LED light 14 are preferably attached to the distal end of the first elongated body 11.

As shown in FIG. 2, the hub 15 is joined to the proximal end of the first elongated body 11. The hub 15 has a base end (proximal) portion 151 on the proximal side, and an indication section 152 on the distal side. The base end portion 151 is provided with the image port 15a, the pressure supply port 15b, and the light supply port 15c as above-mentioned. A cable for connection between the CCD camera 13 and the display device D is led out through the image port 15a, whereas a cable for connection between the LED light 14 and the light source device L is led out through the light supply port 15c. Besides, a tube for injecting into the catheter 1 a liquid or the like supplied from the pressure supply device P may be joined to the pressure supply port 15b.

As shown in FIG. 2, the indication section 152 is provided on its outer peripheral surface with a marker M for precise positioning (alignment) of the balloon 12 in a nasal cavity. The marker M is a scale having scale marks arranged at regular intervals along the extending direction of the catheter 1. The operator moves the catheter 1 forward and backward while reading through the marker M the position of the opening portion of the patient's nasal cavity, whereby precise positioning (alignment) of the balloon 12 in the nasal cavity can be carried out. Incidentally, it is preferable for the indication section 152 provided with the marker M to be movable along the extending direction of the first elongated body 11 and to be fixable relative to the first elongated body 11 at an arbitrary position in the extending direction.

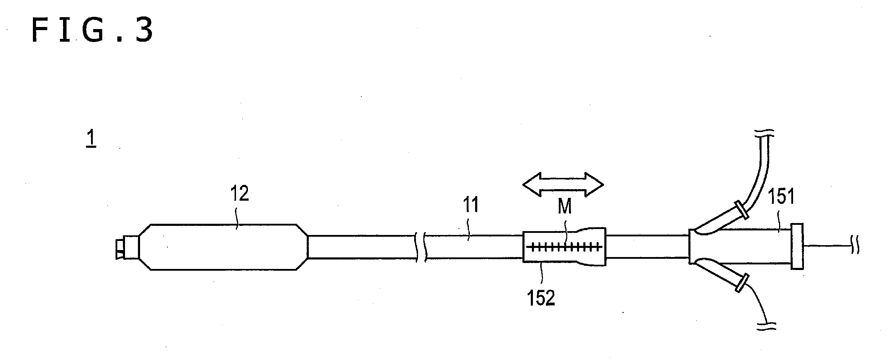

FIG. 3 is a schematic view illustrating a condition wherein the indication section 152 of the catheter 1 of FIG. 2 has been moved along the first elongated body. As shown in FIG. 3, the indication section 152 can be slid at its inner peripheral surface on the outer peripheral surface of the first elongated body 11, whereby it can be moved along the extending direction of the first elongated body 11 (in the directions of arrows in the figure). In addition, the indication section 152 can be tentatively fixed in position relative to the first elongated body 11 by a frictional force acting between its inner peripheral surface and the outer peripheral surface of the first elongated body 11. This ensures that the graduations of the marker M can be made perfectly coincident with the opening portion of the nasal cavity, so that more accurate positioning (alignment) of the balloon 12 can be achieved.

Incidentally, the indication section 152 may be provided with any of various fixing devices for fixing the position thereof relative to the first elongated body 11 more assuredly. Further, the indication section 152 may be in the shape of a clip for gripping of the outer peripheral surface of the first elongated body 11, and may be detachably mounted on the first elongated body 11. This ensures that the indication section 152 can be detached from the first elongated body 11 before or after the positioning (alignment) of the balloon 12, so that usability of the catheter 1 can be enhanced.

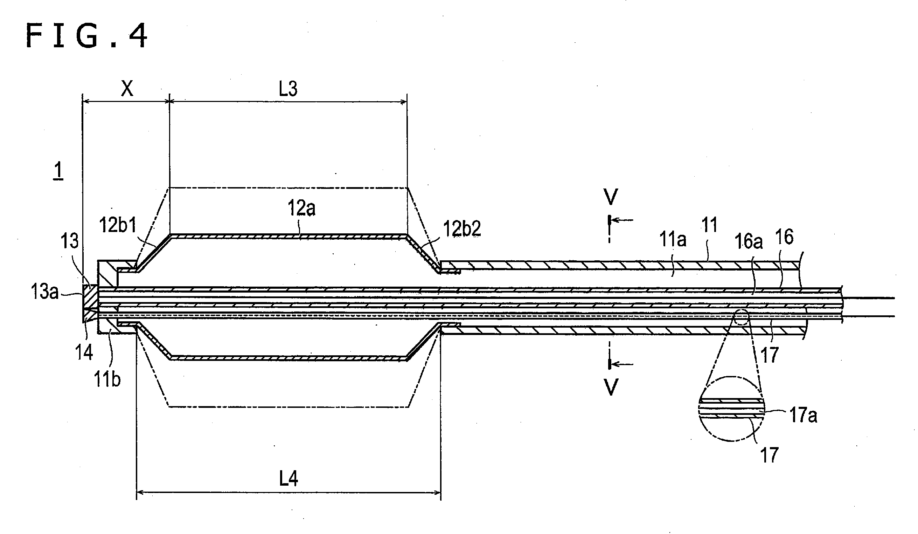

The marker M may be formed by direct printing of a scale on the outer peripheral surface of the indication section 152. Or, alternatively, the marker M may be formed by a method in which a transparent film or the like with scales preliminarily printed thereon is adhered to the outer peripheral surface of the indication section 152 by an adhesive or the like. The scale preferably has graduations for indicating the distance ("X" in FIG. 4) from the image obtaining plane 13a of the CCD camera 13 described later to the distal end of an effective expansive section 12a. Besides, the whole length of the marker M may be set equal to the distance ("X" in FIG. 4). This makes it possible to more accurately position the balloon 12. Incidentally, the form of the scale is not particularly restricted, insofar as it permits the operator to easily read the position of the opening portion of the nasal cavity. Apart from the rectilinear scale shown in the figures, other scales can also be used such as a scale in a lattice form having a predetermined width or a scale in the form of multiple rings extending along the whole circumference of the indication section 152.

FIG. 4 is an enlarged illustration of a longitudinal sectional view showing the vicinity of the distal end of the catheter 1 of FIG. 2. As shown in FIG. 4, the first elongated body 11 has a first lumen 11a, which communicates with the pressure supply port 15b at the proximal end thereof. Therefore, a liquid or the like supplied from the pressure supply device P is injected through the pressure supply port 15b into the first lumen 11a. Injection of a pressurizing medium such as a liquid into the balloon 12 and discharge of the pressurizing medium from the balloon 12 are carried out, whereby expansive deformation and contractive deformation of the balloon 12 can be effected, as required. Incidentally, the pressure of the liquid or the like supplied by the pressure supply device P in the present embodiment is preferably 1 to 30 atm. In addition, the first lumen 11a is not communicating with the image port 15a or the light supply port 15c, so that the liquid or the like injected into the first lumen 11a would not leak to the exterior through the proximal side of the first elongated body 11.

Incidentally, the expansion body is not restricted to the balloon, but may be stent-type expansion bodies formed from a metal with shape-memory characteristics to exert a predetermined pressing force (expansive pressure), for example.

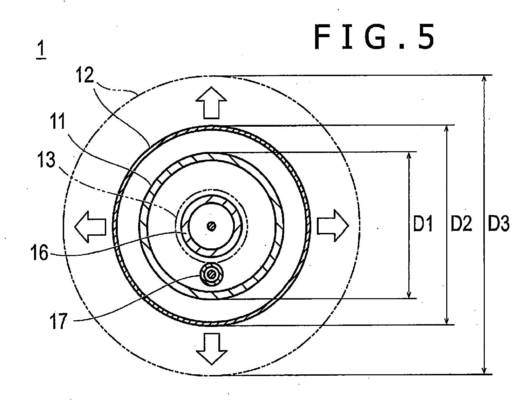

As shown in FIG. 4, the balloon 12 includes a straight barrel-shaped effective expansive section 12a disposed coaxially with the first elongated body 11, and tapered sections 12b1, 12b2 disposed respectively on both sides of the effective expansive section 12a so as to hold the effective expansive section 12a therebetween. The tapered section 12b1 on the distal side is joined airtight to the inner periphery of the first elongated body 11 at the distal end thereof, whereas the tapered section 12b2 on the proximal side is joined airtight to the inner periphery of the first elongated body 11 at the proximal end thereof. Incidentally, the shapes of the distal-side tapered section 12b1 and the proximal-side tapered section 12b2 are preferably in left-right symmetry, with the effective expansive section 12a as a center of symmetry. The balloon 12 is expanded in the radial direction of the effective expansive section 12a by the liquid or the like injected into the first lumen 11a through the pressure supply port 15b. Therefore, when the balloon 12 inserted in a stenosed part of the natural ostium is expanded by the liquid or the like, the outer periphery surface of the effective expansive section 12a presses against the inner peripheral surface of the stenosed part, thereby forcing open the stenosed part. The chain lines in FIG. 4 represent the external shape of the balloon 12 in the expanded state (the same applies also in FIG. 5).

As shown in FIG. 4, the first elongated body 11 is provided at the distal end thereof with an end wall 11b orthogonal to the extending direction thereof, and the distal end of the first lumen 11a is sealed airtight by the end wall 11b. Therefore, the liquid or the like injected into the first lumen 11a would not leak to the exterior through the distal side of the first elongated body 11. In addition, a second elongated body 16 and a third elongated body 17 which are parallel to the first elongated body 11 are disposed inside the first lumen 11a. The second elongated body 16 has a second lumen 16a, and the third elongated body 17 has a third lumen 17a.

As shown in FIG. 4, the vicinity of the distal end of the second elongated body 16 is fitted airtight to the end wall 11a of the first elongated body 11. In addition, the vicinity of the proximal end of the second elongated body 16 is fitted airtight to a base end portion 151 of the hub 15, and the proximal end of the second lumen 16a communicates with the image port 15a. Besides, a cable for connection between the CCD camera 13 and the display device D is contained in the second lumen 16a. This cable is led out to the exterior of the catheter 1 through the image port 15a.

Similarly, the vicinity of the distal end of the third elongated body 17 is fitted airtight to the end wall 11b of the first elongated body 11. In addition, the vicinity of the proximal end of the third elongated body 17 is fitted airtight to the base end portion 151 of the hub 15, and the proximal end of the third lumen 17a communicates with the light supply port 15c. Besides, a cable for connection between the LED light 14 and the light source device L is contained in the third lumen 17a. This cable is led out to the exterior of the catheter 1 through the light supply port 15c.

FIG. 5 is a cross-sectional view taken along line V-V of FIG. 4. As shown in FIG. 5, the radial-directionally center position of the balloon 12 and the radial-directional center position of the first elongated body 11 are preferably disposed coaxially with each other. This ensures enhanced penetrability into the nasal cavity of the balloon 12 before expansion thereof. Similarly, the center position of the CCD camera 13 and the center position of the first elongated body 11 are preferably disposed coaxially with each other. This makes it possible to secure a favorable visual field of the CCD camera 13 on the forward (distal) side of the image obtaining plane 13a.

Now, materials for forming parts of the catheter 1 will be described below. First, the material for the first elongated body 11 will be described. The first elongated body 11 is formed from one of those various resin materials which are generally used in the field of medical catheters. The material should have such a degree of flexibility as to be freely bendable according to a pressure exerted from the inner wall of the nasal cavity without damaging the surrounding mucous membranes when inserted in the nasal cavity. Specific examples of the material which can be used to form the first elongated body 11 include resins such as polyolefins such as polyethylene, polypropylene, ethylene-propylene copolymer, ethylene-vinyl acetate copolymer, and their cross-linked products and partially cross-linked products (e.g., crosslinked ethylene-vinyl acetate copolymer), polyvinyl chloride, nylon elastomer, fluorine resins, polyurethane, etc., and rubbers such as silicone rubbers, latex rubbers, etc.

Incidentally, the second elongated body 16 and the third elongated body 17 should also have such degree of flexibility as to be freely bendable according to a pressure exerted from the inner wall of the nasal cavity without damaging the surrounding mucous membranes when inserted in the nasal cavity, and they are formed from a material similar to the materials for the first elongated body 11. The first to third long bodies may be formed from the same material or from different materials.

Now, the material for the balloon 12 will be described below. The balloon 12 is formed from one of those various resin materials which are generally used in the field of stent delivery systems. Specific examples of the material for the balloon 12 include: polyamides such as homopolymers such as polytetramethyleneadipamide (nylon 46), polycaprolactam (nylon 6), polyhexamethyleneadipamide (nylon 66), polyhexamethylenesebacamide (nylon 610), polyhexamethylenedodecamide (nylon 612), polyundecanolactam (nylon 11), polydodecanolactam (nylon 12), etc. and copolymers such as caprolactam/lauryllactam copolymer (nylon 6/12), caprolactam/aminoundecanoic acid copolymer (nylon 6/11), caprolactam/ω-aminononanoic acid copolymer (nylon 6/9), caprolactam/hexamethylenediammonium adipate copolymer (nylon 6/66), a copolymer of adipic acid with methaxylenediamine, a copolymer of hexamethylenediamine with m,p-phthalic acid, etc.; polyolefins such as polyalkylene resins such as polyethylene resins such as linear low-density polyethylene (LLDPE), low-density polyethylene (LDPE), and high-density polyethylene (HDPE), and polypropylene resins, etc., ethylene-propylene copolymer, ethylene-vinyl acetate copolymer, and their crosslinked products and partially crosslinked products (e.g., crosslinked ethylene-vinyl acetate copolymer), etc.; epoxy resins, urethane resins, diallyl phthalate resins (allyl resins), polycarbonate resins, fluorine resins, amino resins (urea resin, melamine resin, benzoquanamine resin), polyester resins (e.g., polyethylene terephthalate), styrol resins, acrylic resins, polyacetal resins, vinyl acetate resin, phenolic resins, vinyl chloride resin, silicone resins (silicon resins), polyarylene sulfides (e.g., polyphyenylene sulfide); silicone rubbers, latex rubbers; and nylon elastomers as block copolymers of nylon 6, nylon 66, nylon 11, nylon 12, or the like as a hard segment with polyalkylene glycol, polyether, an aliphatic polyester or the like as a soft segment.

These materials may be used either singly or in combination of two or more of them. In addition, the material is used in the form of a monolayer film or a multilayer film. Besides, a synthesized product of the above-mentioned polymeric material may be used, or a commercial product of the polymeric material may also be used.

Now, dimensions of various parts of the catheter 1 will be described below. Since the catheter 1 is used in a method of treating sinusitis as described later, the dimensions of various parts of the catheter 1 are preferably in ranges suitable for insertion of the catheter 1 into a nasal cavity. Specifically, the whole length ("L1" in FIG. 2) of the first elongated body 11 is preferably in the range of 50 to 500 mm. In addition, the outer diameter ("D1" in FIG. 5) of the first elongated body 11 is preferably 0.3 to 3 mm. The whole length ("L2" in FIG. 2) of the marker M provided at the hub 15 serving as a proximal operating section is preferably 5 to 490 mm. Incidentally, the dimensions of the CCD camera 13, the LED light 14, the hub 15, the second elongated body 16, the third elongated body 17 and the like are appropriately determined according to the dimensions of the first elongated body 11.

Now, the dimensions of the balloon 12 will be described below. The whole length ("L3" in FIG. 4) of the effective expansive section 12a of the balloon 12, preferably, is sufficiently greater than the whole length of a general stenosed part of the natural ostium in the patient of sinusitis; specifically, it is preferably 5 to 30 mm. The whole length ("L4" in FIG. 4) of the balloon 12 inclusive of the tapered sections 12b1, 12b2 is preferably 32 to 60 mm in the case where the whole length ("L3" in FIG. 4) of the effective expansive section 12a is 30 mm. Besides, the distance ("X" in FIG. 4) from the image obtaining plane 13a of the CCD camera 13 to the distal end of the effective expansive section 12a is preferably 2 to 20 mm.

In addition, the outside diameter ("D2" in FIG. 5) of the balloon 12 before expansion is preferably 0.3 to 5 mm, for ease of passage through the stenosed part of the natural ostium. The outside diameter ("D3" in FIG. 5) after expansion, preferably, is substantially equivalent to an ideal outside diameter of the communicating passage between the nasal cavity and the paranasal sinus to be formed at the natural ostium in the patient of sinusitis; specifically, it is preferably 2 to 10 mm.

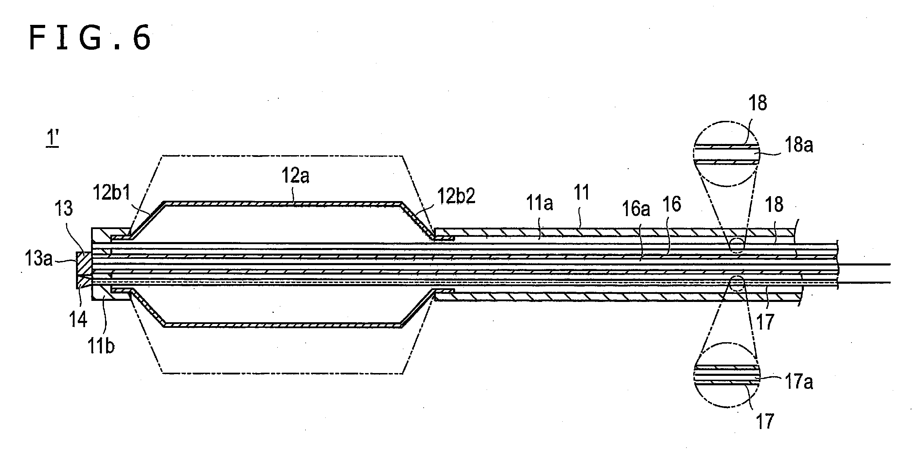

Now, a catheter 1' as a modification of the catheter 1 according to the present embodiment will be described below. FIG. 6 is an enlarged illustration of a longitudinal sectional view showing the vicinity of the distal end of the catheter 1'. As shown in FIG. 6, the catheter 1' has a fourth elongated body 18 in addition to the components of the catheter 1 described above. The catheter 1' is the same in structure as the above-described catheter 1, except for the parts relating to the fourth elongated body 18. In the following, therefore, the components of the catheter 1' are denoted by the same reference symbols as those used above for the components of the catheter 1.

As shown in FIG. 6, in addition to the second elongated body 16 and the third elongated body 17, a fourth elongated body 18 having a fourth lumen 18a is disposed in the first lumen 11a of the first elongated body 11. In addition, the vicinity of the distal end of the fourth elongated body 18 is fitted airtight to the end wall 11a of the first elongated body 11, while the vicinity of the proximal end of the fourth elongated body 18 is fitted airtight to the base end portion 151 of the hub 15. In other words, the fourth lumen 18a communicates with the exterior of the catheter 1' at both ends thereof. A guide wire for guiding the catheter 1' to a desired part inside the nasal cavity is inserted and passed through the fourth lumen 18a. This point will be further described later.

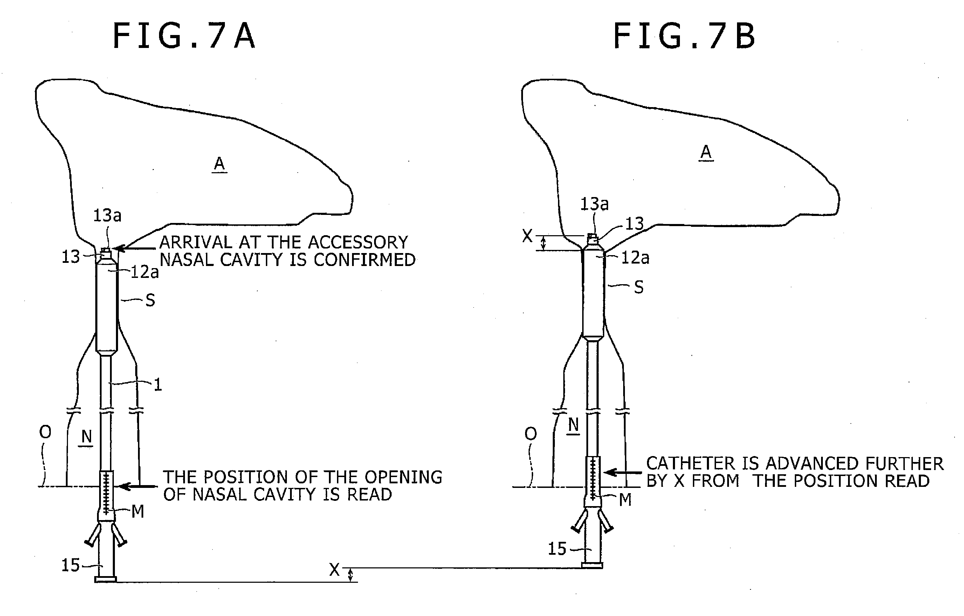

Now, a method of positioning (aligning) the balloon 12 using the marker M provided on the hub 15 (indication section 152) serving as the proximal operating section will be described below referring to FIGS. 7A and 7B. First, in Step (1-1), the operator advances the catheter 1 inside a nasal cavity N while watching the image obtained by the CCD camera 13 and displayed on the display device D, and then stops the catheter 1 when an image of the inside of the paranasal sinus (paranasal sinus) A is confirmed on the display device D. By this step, the CCD camera 13 can be passed through a stenosed part S of the natural ostium, and the imaging surface 13a can be made to arrive at the entrance of the paranasal sinus A. FIG. 7A is a schematic view showing the positional relationship between the nasal cavity N and the catheter 1 upon completion of Step (1-1).

Next, in Step (1-2), the operator reads the graduation on the marker M that indicates the position of the nasal cavity opening O upon completion of Step (1-1). In this case, it is preferable to move the indication section 152 along the first elongated body 11, as required, so that the graduation on the marker M agrees perfectly with the nasal cavity opening O. Then, in Step (1-3), the operator advances the catheter 1 further by a length equal to the distance (see "X" in FIG. 4) between the effective expansive section 12a of the balloon 12 and the imaging surface 13a of the CCD camera 13, from the graduation on the marker M that is read in Step (1-2). Consequently, the distal end of the effective expansive section 12a can be made to arrive at the entrance of the paranasal sinus A. FIG. 7B is a schematic view showing the positional relationship between the nasal cavity N and the catheter 1 upon completion of Step (1-3).

Since the effective expansive section 12a of the balloon 12 can be disposed over the whole length of the stenosed part S by the above-described method as shown in FIG. 7B, the stenosed part S can entirely be forced open when the balloon 12 is expanded. Therefore, according to the method, the stenosed part S of the natural ostium can be forced open more assuredly, so that a therapeutic effect on sinusitis can be enhanced. In addition, it is possible by the above-described method to effectively prevent the catheter 1 from being excessively advanced into the paranasal sinus, and, therefore, safety in treatment of sinusitis can be enhanced.

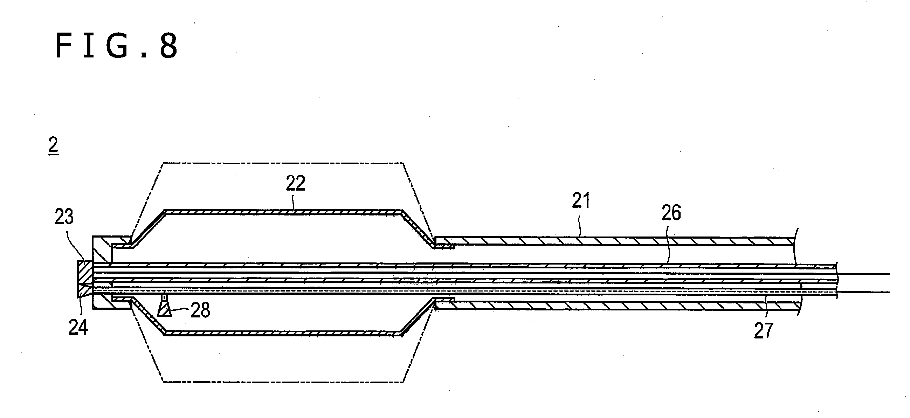

Now, a catheter 2 as a treatment device according to a second embodiment of the present invention will be described below. FIG. 8 is an enlarged illustration of a longitudinal sectional view showing the vicinity of the distal end of the catheter 2 according to the present embodiment. As shown in FIG. 8, the catheter 2 includes a first elongated body 21, a balloon 22, a CCD camera 23, an LED light 24, a hub 25 (not shown), a second elongated body 26, and a third elongated body 27, like the catheter 1 according to the first embodiment described above. The structure of various parts of the catheter 2 is similar to the first embodiment above, except for those specified below. It is to be noted here, however, that a light-permeable material, selected from the above-mentioned materials for the balloon 12, is used for forming the balloon 22.

As shown in FIG. 8, the catheter 2 has a second LED light 28 disposed inside the balloon 22, in addition to the LED light 24 attached to the first elongated body 21. For distinction from the second LED light 28, in the following, the LED light 24 will be referred to as "the first LED light 24." As will be described later, the second LED light 28 is used for precise positioning (alignment) of the balloon 22 inside a nasal cavity, as a substitute for the marker M in the first embodiment above or as a complement to the marker M.

As shown in FIG. 8, the second LED light 28 is mounted on a third elongated body 27, and is connected to the light source device (not shown) in the exterior through a cable inside the third lumen 27a, like the first LED light 24. The second LED light 28 is so disposed as to be able to illuminate the radially outer side of the balloon 22 through the balloon 22, which is made of a light-permeable material. Incidentally, the number and layout of the second LED light(s) 28 is not particularly limited, insofar as the radially outer side of the balloon 22 can be illuminated thereby. For instance, a plurality of the second LED lights 28 may be disposed along the third elongated body 27. In addition, the structure in which the second LED light 28 is disposed inside the balloon 22 as in the example shown in FIG. 8 is not restrictive, and the second LED light may be embedded in the balloon 22 itself.

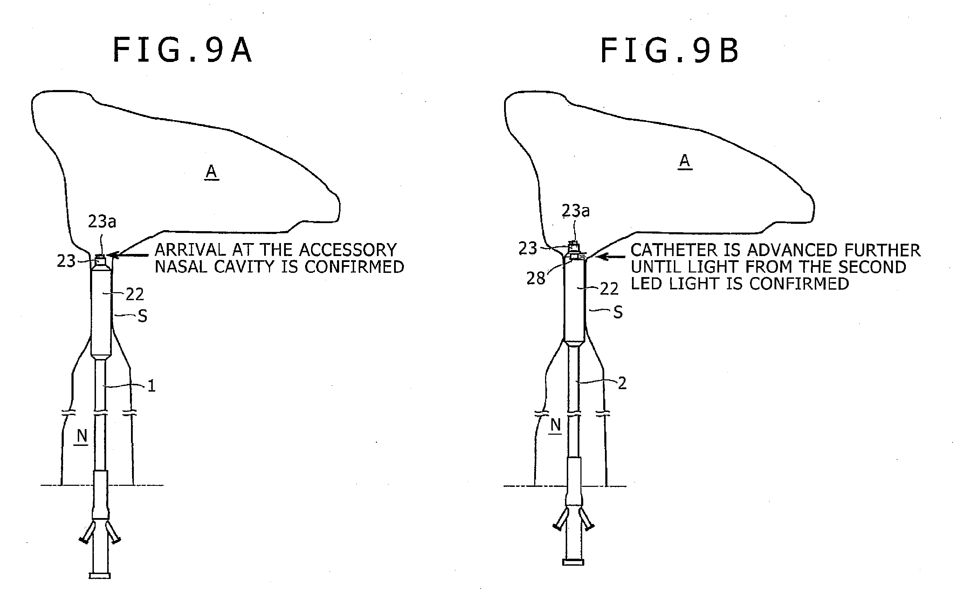

Now, a method of positioning (aligning) the balloon 22 using the above-mentioned second LED light(s) 28 will be described referring to FIGS. 9A and 9B. First, in Stet (2-1), the operator advances the catheter 2 inside the nasal cavity N while checking the image obtained by the CCD camera 23 and displayed on the display device D, and stops the catheter 2 upon confirmation of an image of the inside of the paranasal sinus A on the display device D. By this Step, the CCD camera 23 can be passed through a stenosed part S of the natural ostium, and the imaging surface 23a can be made to arrive at the entrance of the paranasal sinus A. FIG. 9A is a schematic view showing the positional relationship of the catheter 2 relative to the nasal cavity N upon completion of Step (2-1).

Subsequently, in Step (2-2), the operator turns off the first LED light 24, and turns on the second LED light 28 instead. Then, in Step (2-3), the operator advances the catheter 2 further inside the nasal cavity N while checking the image obtained by the CCD camera 23 and displayed on the display device D, and stops the catheter 2 upon confirmation of the light from the second LED light 28 on the display device D. By this Step, not only the CCD camera 23 but also the vicinity of the distal end of the balloon 22 can be advanced into the paranasal sinus A. FIG. 9B is a schematic view showing the positional relationship of the catheter 2 relative to the nasal cavity N upon completion of Step (2-3).

As shown in FIG. 9B, it is possible by the above-described method to dispose the balloon 22 over substantially the whole length of the stenosed part S, so that the stenosed part S can entirely be forced open when the balloon 22 is expanded. According to the method, therefore, the stenosed part S of the natural ostium can be forced open more assuredly, so that a therapeutic effect on sinusitis can be enhanced. In addition, it is possible by the method to effectively prevent the catheter 2 from being excessively advanced into the paranasal sinus, so that safety in treatment of sinusitis can be enhanced.

### <METHOD OF TREATING SINUSITIS BY USE OF TREATMENT DEVICE ACCORDING TO THE PRESENT INVENTION>

As has been described above, the treatment device according to the present invention is suitably applicable to treatment of sinusitis. Here, the form in which the treatment device of the present invention is used for treatment of sinusitis is not specifically restricted. For instance, the treatment device according to the present invention is introduced into a nasal cavity, and, when the entrance of the paranasal sinus (paranasal sinus) is confirmed by the imaging unit provided at the distal side on the treatment device, the expansion body is expanded to dilate a stenosed part of the paranasal sinus. Thus, the present invention provides a method of treating sinusitis, including: (i) introducing into a nasal cavity a flexible elongated body which integrally has an expansion body provided on the elongated body and imaging unit provided on the distal side relative to the expansion body; (ii) confirming the entrance of a paranasal sinus based on an image obtained by the imaging unit; (iii) expanding the expansion body so as to dilate a stenosed part of a natural ostium between the nasal cavity and the paranasal sinus. Incidentally, the term "paranasal sinus" herein may be any of frontal sinus, ethmoidal sinus, sphenoidal sinus, and maxillary sinus.

According to the just-mentioned method, it is possible to easily and accurately position (align) the expansion body inside the nasal cavity and to dilate a stenosed part of the paranasal sinus assuredly and easily, by use of a simple device such as an endoscope, without using a special device such as a radioscopic device. In addition, the method according to the present invention is a minimally invasive method by means of a catheter, and, therefore, it exerts little burden on the patient.

Now, preferred embodiments of the method of treating sinusitis by use of the treatment device according to the present invention will be described referring to the drawings. Incidentally, the present invention is not to be restricted to the following embodiments.

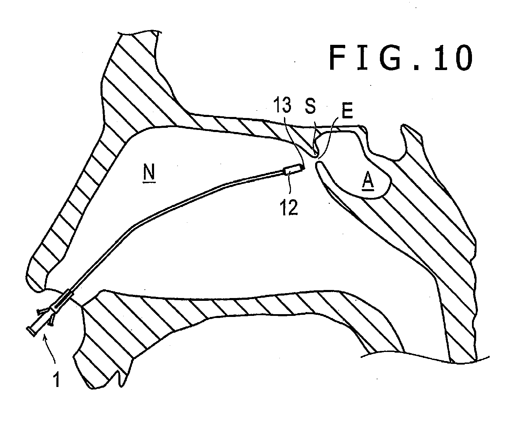

Referring to FIG. 10, first, the catheter (treatment device) 1 is advanced through a naris and through a nasal cavity N into the vicinity of the entrance E of the paranasal sinus A (for example, the sphenoidal sinus shown in FIG. 10).

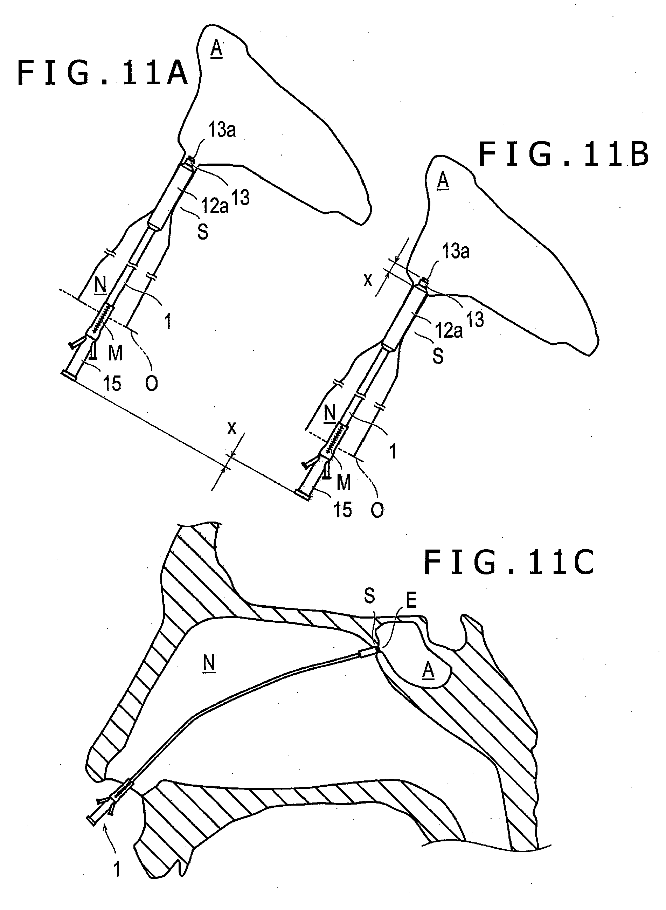

Referring to FIGS. 11A and 11C, after the entrance E of the paranasal sinus A is confirmed based on an image obtained by the CCD camera 13, the forward movement of the catheter 1 is stopped and the catheter 1 is tentatively disposed.

In this instance, the position of the catheter 1 in the living body can be confirmed through the function of the CCD camera 13. In addition, the position of the entrance E of the paranasal sinus A is confirmed by confirming the advance of the CCD camera 13 into the paranasal sinus A on the basis of the image obtained by the CCD camera 13.

After the image of the inside of the paranasal sinus A is confirmed on the display device D, the catheter 1 is stopped. With the image of the inside of the paranasal sinus A obtained, it can be confirmed that the image obtaining plane 13a of the CCD camera 13 is passed through the stenosed part S of the natural ostium to arrive at the entrance of the paranasal sinus A.

Referring to FIG. 11B, next, the graduation on the marker M that indicates the position of the nasal cavity opening O is read, and, while observing the graduation, the catheter 1 is advanced further by a length equal to the distance (refer to "X" in FIG. 4) between the effective expansive section 12a of the balloon 12 and the image obtaining plane 13a of the CCD camera 13. By this step, the distal end of the effective expansive section 12a can be made to arrive at the entrance E of the paranasal sinus A.

Thus, in the method according to the present invention, it is preferable that after Step (ii) (confirmation of the position of the catheter 1), the elongated body 11 is advanced further from the position confirmed in Step (ii), by a length equal to the distance between the effective expansive section 12a (of the expansion body 12) for pressing the stenosed part S and the image obtaining plane 13a of the imaging unit 13, before the step of expanding the expansion body 12 is performed.

In the case of using a catheter 2 shown in FIG. 8, the introduction of the catheter 2 can be carried out using the first LED light 24 for illuminating the distal end of the first elongated body 11 and the second LED light 28 for illuminating the distal end of the balloon 12.

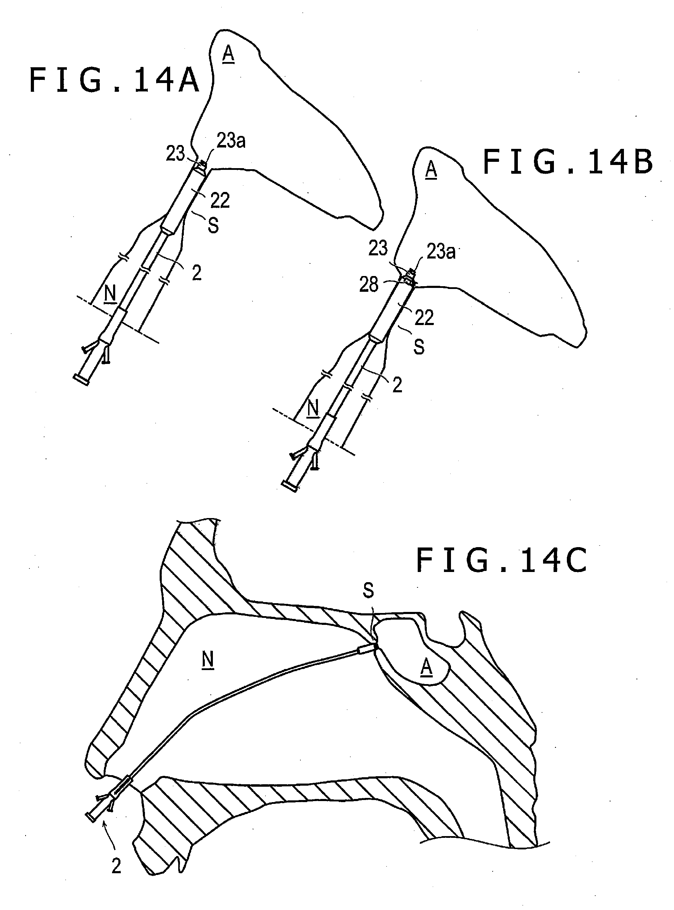

Referring to FIGS. 14A and 14C, the catheter 2 is advanced within a nasal cavity N while keeping the first LED light 24 on, and the catheter 2 is stopped when an image of the inside of the paranasal sinus A is confirmed on the display device D. It is confirmed that the image obtaining plane 23a of the CCD camera 23 has been passed through a stenosed part S of the natural ostium, to reach the entrance E of the paranasal sinus A.

Referring to FIG. 14B, the first LED light 24 is turned off, and the second LED light 28 is turned on instead. In this instance, though the second LED light 28 illuminates the natural ostium between the nasal cavity N and the paranasal sinus A, but the light does not reach the paranasal sinus A. Since the paranasal sinus A is a dark place, the paranasal sinus A cannot be confirmed based on the image obtained by the CCD camera 23.

Next, while observing the image obtained by the CCD camera 23 and displayed on the display device D, the catheter 2 is advanced further within the nasal cavity N. Upon confirmation of the light emitted from the second LED light 28 on the display device D, the catheter 2 is stopped. By this step, not only the CCD camera 23 but also a portion near the distal end of the balloon 22 can be advanced into the paranasal sinus A.

Incidentally, in the present invention, the treatment device (catheter 1, 2) according to the present invention may be advanced directly through the naris and the nasal cavity N into the vicinity of the entrance E of the paranasal sinus A, but this is not restrictive. Alternatively, the treatment device 1, 2 may be disposed into the vicinity of the entrance E of the paranasal sinus A by use of an assisting device such as a guide catheter or a guide wire.

An example of the method of introducing the treatment device (catheter 1, 2) by use of a guide catheter or a guide wire will be described in brief.

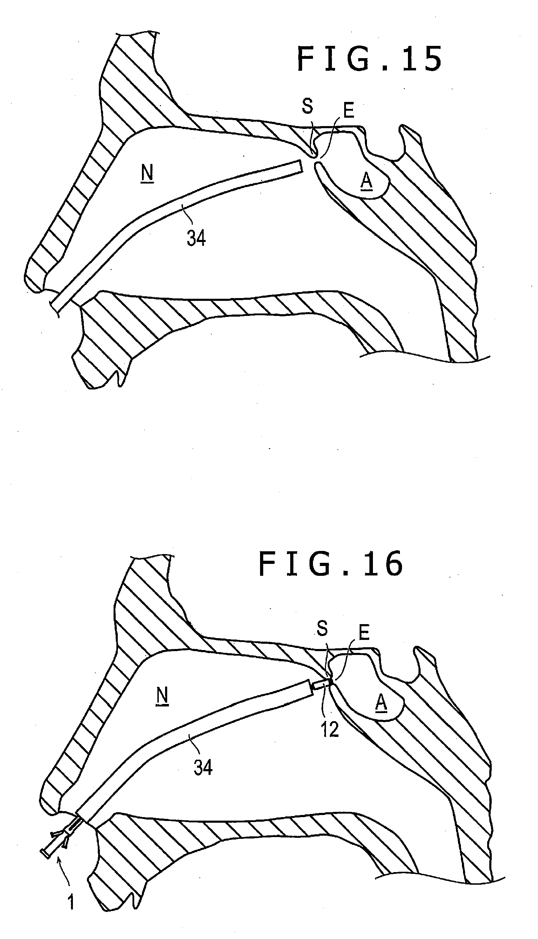

Referring to FIG. 15, the guide catheter 34 is introduced via the naris, and is advanced through the nasal cavity N into the vicinity of the entrance E of the paranasal sinus A.

Referring to FIG. 16, after the guide catheter 34 is advanced to an appropriate position, the catheter 1 can be advanced through the guide catheter 34 into the vicinity of the entrance E of the paranasal sinus A.

The guide catheter 34 to be used in introducing the catheter 1 is not specifically restricted, and the same or equivalent guide catheters to those commonly used in the medical field can be similarly used. Taking operability and less-invasiveness to nasal cavity into consideration, however, a flexible guide catheter is preferred. Specific examples of the material which can be used to form the guide catheter, therefore, include: such polymers as polyimides, polyurethane, nylon, polyvinyl chloride (PVC), high-density polyethylene (HDPE), polyether ether ketone (PEEK), etc.; fluorinated polymers such as polytetrafluoroethylene (PTFE), tetrafluoroethylene-perfluoroalkyl vinyl ether copolymer (PFA), tetrafluoroethylene-hexafluoropropylene copolymer (FEP), tetrafluoroethylene-ethylene copolymer (ETFE), etc.; and metals such as stainless steel. In addition, the diametral size (thickness) and length of the guide catheter 34 are not particularly limited but may be appropriately selected according to the weight and body type of the patient. The guide catheter 34 may have its surface coated with an appropriate coating such as a surface lubricating coating, for favorable sliding thereof.

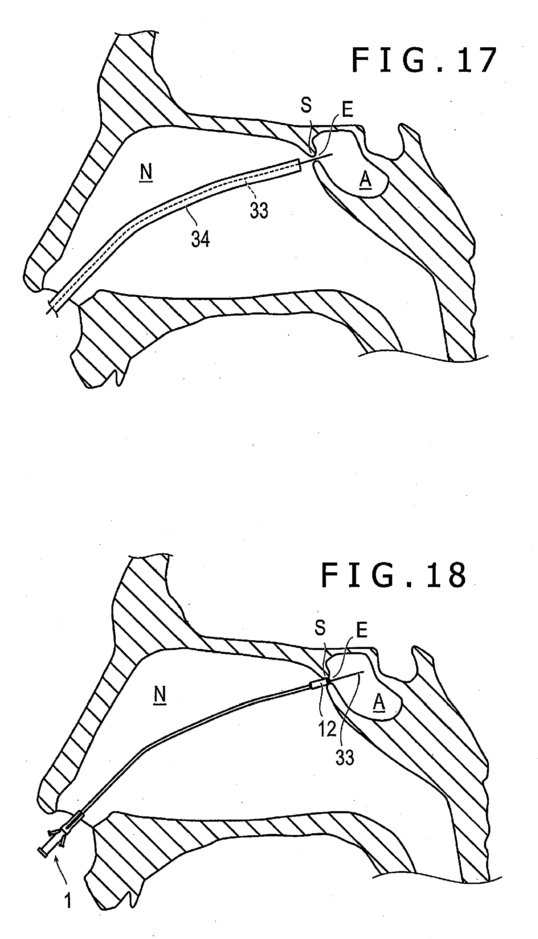

Referring to FIG. 17, after the guide catheter 34 is advanced into the vicinity of the entrance E of the paranasal sinus A, the guide wire 33 can be introduced through the guide catheter 34 into the paranasal sinus A. In the case of using the guide wire 33, the catheter 1' shown in FIG. 6 is used. The catheter 1' is provided with the fourth elongated body 18 having the lumen 18a through which the guide wire 33 is inserted and passed.

The guide wire 33 to be used for the introduction is not specifically restricted, and the same or equivalent guide catheters to those commonly used in the medical field can be used similarly.

Referring to FIG. 18, after the guide catheter 34 is evulsed from the guide wire 33, the guide wire 33 is inserted into and passed through the lumen 18a of the catheter 1', and the catheter 1' is advanced along the guide wire 33 into the vicinity of the entrance E of the paranasal sinus A.

Incidentally, only the guide wire 33 is advanced into the vicinity of the entrance E of the paranasal sinus A without using the guide wire 34, and then the catheter 1' is advanced along the guide wire 33 into the vicinity of the entrance E of the paranasal sinus A.

Now, an operation of dilating a stenosed part S of the natural ostium will be described below.

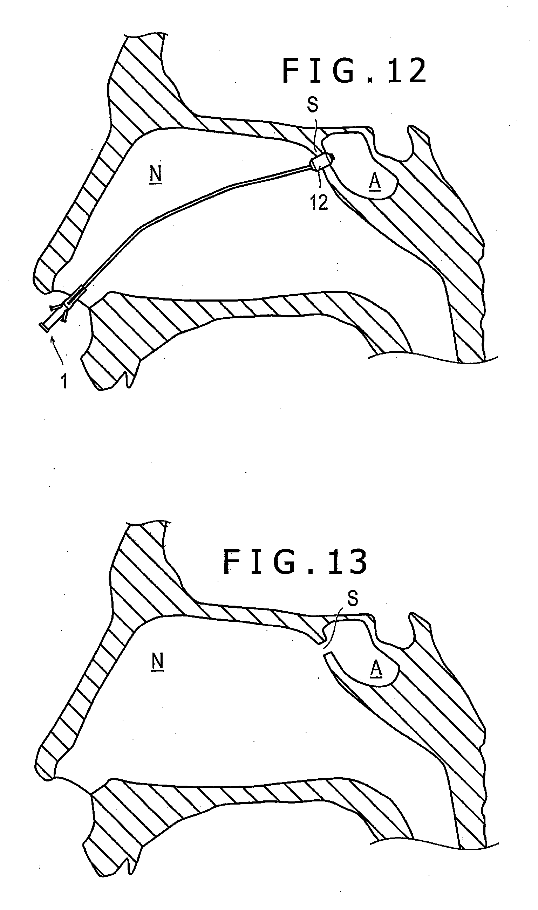

Referring to FIG. 12, after the distal end of the effective expansive section 12a of the balloon 12 provided on each of the catheters 1, 1' and 2 (in the following descriptions, the catheter 1 will be taken as a representative of the catheters), the balloon 12 is expanded. Attendant on the expansion, the balloon 12 exerts a pressure on the stenosed part S of the natural ostium between the nasal cavity N and the paranasal sinus A, to dilate the stenosed part S.

The expansion of the balloon 12 may be carried out by use of a medium, which may be the same or equivalent to those commonly used in the medical field. Specific examples of the medium which can be used here include gases such as air, nitrogen, carbon dioxide, etc.; and liquids such as physiological saline, a radiopaque material. Besides, the amount of the medium injected into the expansion body is not particularly limited but may be appropriately selected according to the inside diameter of the stenosed part S (the inside diameter of the natural ostium), the inside diameter of the natural ostium after dilation, or the like.

The expansion of the balloon 12 can be performed with the catheter 1 being positionally-fixed, or while the elongated body 12 is advanced forward or retreated backward.

According to the method in which the expansion is conducted with the catheter 1 being positionally-fixed, a pressure can be exerted on the stenosed part S more assuredly, so that a smooth and speedy technique can be achieved. On the other hand, the method in which the expansion is conducted while the elongated body 12 is advanced forward or retracted backward can be suitably applied to the case where the stenosed part S has a comparatively long extension along the natural ostium.

After the balloon 12 is thus expanded, the catheter 1 is evulsed. In this instance, the evulsion of the catheter 1 is preferably carried out while confirming that the stenosed part S has been dilated, based on the image obtained by the imaging unit 13. By this step, it can be visually confirmed that the stenosed part S has been dilated securely. In addition, the evulsion of the catheter 1 is preferably performed, for example, with the expansion body 12 contracted, or while expansive deformation and contractive deformation of the expansion body 12 are carried out in an appropriate manner. By such an operation, more assured dilation can be achieved.

Besides, the operation of advancing the catheter 1 beyond the stenosed part S into the vicinity of the entrance of the paranasal sinus A can be conducted concurrently with the operation of expanding the balloon 12. This ensures that the catheter 1 can be advanced while forcing open the stenosed part S, so that the stenosed part S can be dilated more assuredly.

Referring to FIG. 13, after the balloon 12 is expanded, the catheter 1 is evulsed, resulting in that the stenosed part S of the natural ostium between the nasal cavity N and the paranasal sinus A is in a dilated state.

According to the method of the present invention as above-described, the expansion body (balloon) can be disposed over substantially the whole length of the stenosed part, so that the stenosed part can entirely be forced open when the expansion body (balloon) is expanded. This makes it possible to force open the stenosed part of the natural ostium more assuredly, so that the therapeutic effect on sinusitis can be enhanced. In addition, according to the above-described method, the catheter can be effectively prevented from being excessively advanced into the paranasal sinus, so that safety in treating sinusitis can be enhanced.

Furthermore, according to the method of the present invention, it is possible to easily and accurately position (align) the expansion body inside the nasal cavity and to reliably and easily dilate the stenosed part of the paranasal sinus, by use of an existing CCD camera or the like, without using a special device such as a radioscopic device. Besides, the method according to the present invention is a minimally invasive method based on the use of a catheter, so that it exerts little burden on the patient.

Now, a catheter 3 as a treatment device according to a third embodiment of the present invention will be described below.

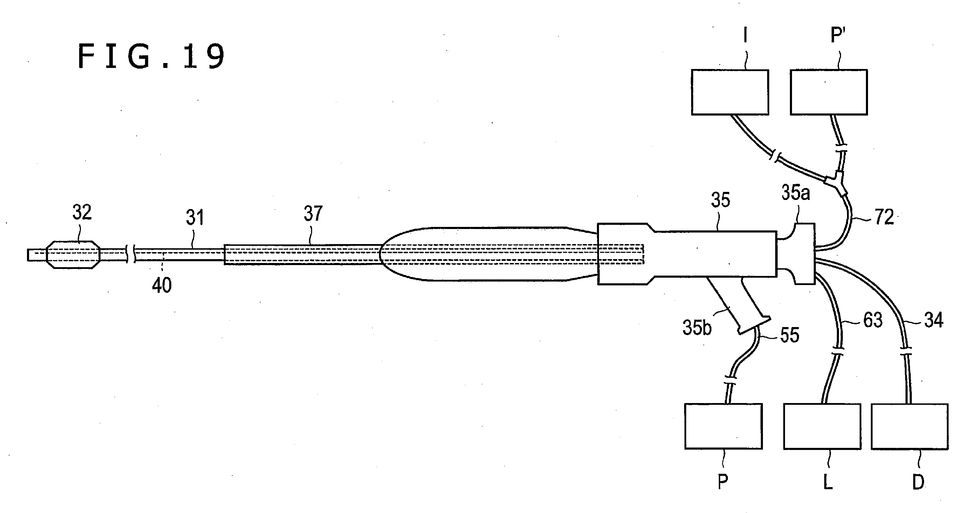

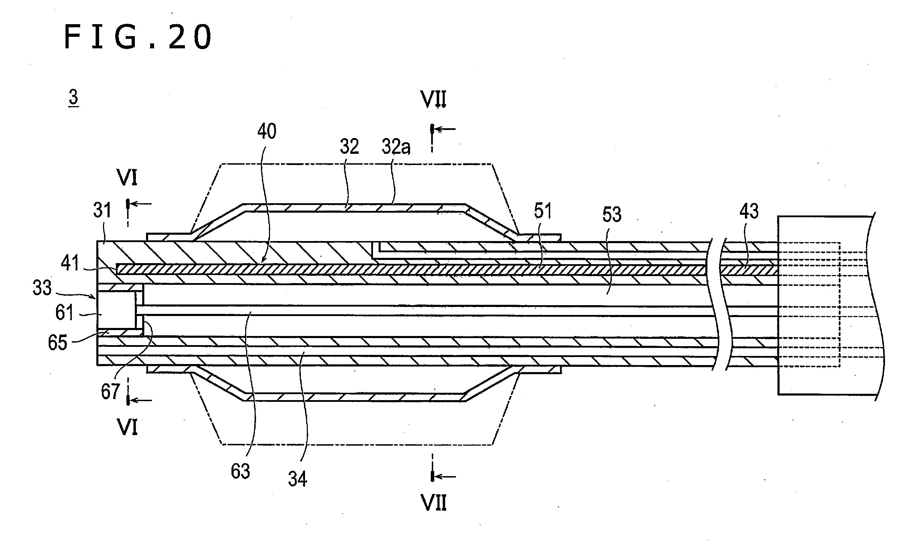

FIG. 19 is a schematic illustration of the general configuration of the catheter 3, and FIG. 20 is a longitudinal sectional view of a major part of the catheter 3. The catheter 3 has a holding member 40 by which the external shape of a first elongated body 31 is held in a predetermined shape. The catheter 3 differs from the catheter 1 in the first embodiment above and the catheter 2 in the second embodiment above, in that it has such a holding member 40. Incidentally; with respect to the same members as those in the above-described embodiments, the descriptions will be partly omitted.

Accessory nasal cavities (paranasal sinuses) include frontal sinus, ethmoidal sinuses, sphenoidal sinus and maxillary sinus, and paths of introduction of the treatment device from the nasal cavity into the individual accessory nasal cavities are different from each other. In treating sinusitis, therefore, for guiding the treatment device into the vicinity of the desired one of the accessory nasal cavities, it may be necessary to prepare a plurality of kinds of treatment devices respectively having product specifications (external shapes) according to the introduction paths and introduction angles for the treatment devices, which needs much labor and cost. In addition, even where the plurality of treatment devices differing in product specifications are prepared, it is difficult to cope also with individual differences in anatomical structure from patient to patient. The catheter 3 according to the present embodiment is so configured that a first elongated body 31 constituting a main body portion of the catheter 3 can be reshaped at the time of introduction into a living body. With the first elongated body 31 thus made to be reshapable, the catheter 3 having the first elongated body 31 as a main body portion thereof can be reshaped. Furthermore, the external shape of the catheter 3 can be maintained in the living body. As a result, the catheter 3 and a balloon 32 can be easily and assuredly guided into the vicinity of a stenosed part of the natural ostium, irrespectively of differences in introduction path to each paranasal sinus to be treated and individual differences in anatomical structure from patient to patient.

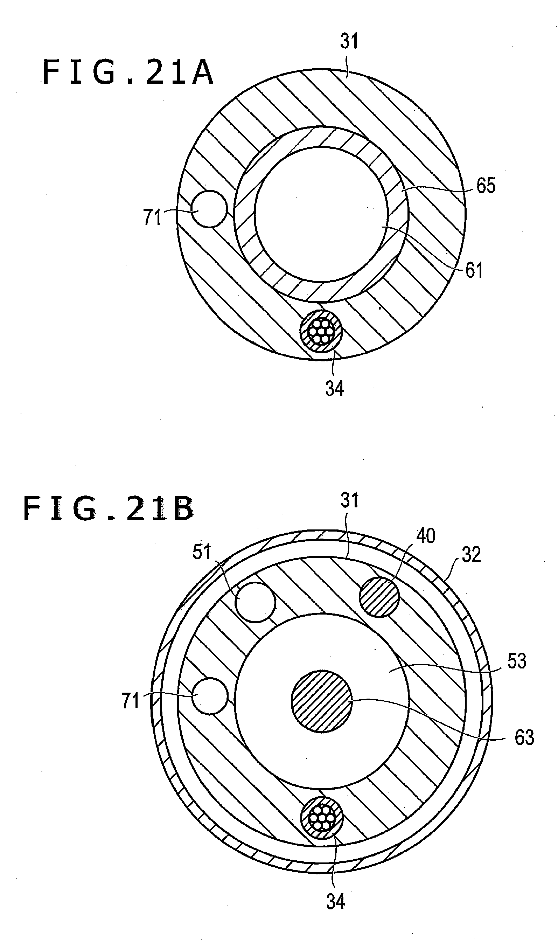

The catheter 3 according to the present embodiment will be described referring to FIGS. 19 to 21B. FIG. 19 is a schematic view showing the general configuration of a treatment system including the catheter 3, FIG. 20 is an enlarged illustration of a longitudinal sectional view showing the vicinity of the distal end of the catheter 3, FIG. 21A is an enlarged sectional view along line VI-VI of FIG. 20, and FIG. 21B is an enlarged sectional view taken along line VII-VII of FIG. 20.

The catheter 3 includes the first elongated body 31 provided with the balloon 32 as an expansion body, a holding member 40 enclosed in the first elongated body 31, an outer tube member 37 so disposed as to cover predetermined portions of the first elongated body 31 and the holding member 40, a hub 35 used as a proximal operating section, and a main port 35a and a pressure supply port 35b which are provided on the hub 35.

Expansion of the balloon 32 is conducted by a method in which a pressurizing medium such as a liquid is fed into a pressurizing medium holding space defined between the balloon 32 and the first elongated body 31, through a pressuring medium lumen 51 disposed in the catheter 3.

A CCD camera 33 includes an image sensor part 61 disposed inside the first elongated body 31, a connection cable 63 for signal transmission/reception which is electrically connected to the image sensor part 61, and a protective member 65 covering the image sensor part 61. The image sensor part 61 covered with the protective member 65 and the connection cable 63 are disposed in a CCD camera lumen 53.

The image sensor part 61 contains precision apparatuses such as optical elements (e.g., camera lens), light receiving elements, and circuit elements for generation and transmission/reception of various signals at the time of obtaining an image. The transmission/reception of signals between the image sensor part 61 and a display device is conducted through the connection cable 63.

The protective member 65 is provided for preventing the image sensor part 61 from being broken. The protective member 65 is formed in a tubular shape, and the image sensor part 61 is housed in the protective member 65. The protective member 65 can protect the image sensor part 61 from external shocks. Besides, the protective member 65 can also prevent the image sensor part 61 from being excessively curved or bent.

The protective member 65 can be formed, for example, from a metallic material such as iron or aluminum. However, the material of the protective member 65 is not restricted to metallic materials, and the material can be changed appropriately insofar as it can protect the image sensor part 61. In addition, the protective member 65 is provided with an opening 67 through which to lead out the connection cable 63 connected to the image sensor part 61.

The lumen 51 for balloon expansion is connected to a pressure supply device P, such as an indeflator, through a predetermined tube 55 and the pressure supply port 35b provided on the hub 35. The CCD camera 33 is electrically connected to a display device D, such as an LCD, through the connection cable 63 and the main port 35a provided on the hub 35. An optical fiber 34 is connected to a light source device L via the main port 35a provided on the hub 35. Incidentally, other lighting units such as an LED lamp, a halogen lamp and a high-intensity discharge lamp (HID lamp) can also be used, in place of the optical fiber.

The first elongated body 31 has a suction lumen 71 for suctioning fluids present in a living body, such as body fluid and various secretions (refer to FIGS. 21A and 21B). The suction lumen 71 is connected to a suction device P' via a predetermined tube 72 and the main port 35a provided on the hub 35. As the suction device P', a known fluid pump ordinarily used for fluid suctioning or the like may be used. Besides, the suction lumen 71 can be used as a lumen for providing fluids, in this case, the suction lumen 71 is connected to fluids send device I through the predetermined tube 72 being connected to the suction device P and being connected to a connector 73.

The holding member 40 has a metallic bar member of a long shape extending in the extending direction of the first elongated body 31. The holding member 40 is composed of a bar member of a metallic material which is comparatively flexible or soft.

The "long shape" possessed by the holding member 40 is a shape extending in the extending direction of the first elongated body 31. With the holding member 40 having such a shape, the catheter 3 as a whole can be deformed so that it is curved into an arbitrary direction crossing the axial direction thereof. In addition, when the first elongated body 31 is curved, the first holding member 40 can prevents the first elongated body 31 from being excessively bent. As a result, the first elongated body 31 can be prevented from being broken, and each of the lumens provided in the first elongated body 31 can be prevented from being greatly changed in inner cavity shape.

Besides, the expression "the holding member is enclosed in the first elongated body" includes a form of the holding member 40 being disposed in a lumen formed inside the first elongated body 31, and a form of the holding member 40 being embedded in the inner wall of the first elongated body 31. Thus, the expression widely means disposition of the holding member 40 in the state of being covered with the first elongated body 31.

On the proximal side of the holding member 40, there is provided a proximal section 43 having a metallic bar member of a material more rigid than the holding member 40.

The holding member 40 can be composed of a bar member formed, for example, from copper, brass or other soft metal. The rigidity of the holding member 40 is set at such a magnitude that the first elongated body 31 introduced into a living body can be thereby held in a predetermined curved shape (an external shape with which at least a part of the first elongated body 31 being curved into a direction crossing the axial direction). Therefore, the rigidity of the holding member 40 is designed to be at least higher than the rigidity of the first elongated body 31. In addition, the rigidity is preferably at such a level that reshaping of the first elongated body 31 can be achieved by a simple manual operation. Accordingly, for example, the rigidity of the holding member 40 is designed to be about 50 to 150 Hv. On the other hand, the rigidity of the first elongated body 31 can be designed appropriately within the range below the rigidity of the holding member 40.

The outer tube member 37 and the proximal section 43 for the holding member 40 can be formed, for example, of a material which is higher than the holding member 40 in rigidity, such as iron, stainless steel, and rigid resin materials. In the example shown in the drawings, the holding member 40 and the proximal section 43 are integrally configured by jointing two metallic bars which are different in rigidity.

The proximal section 43 and the outer tube member 37 are intended to enhance pushability of the catheter 3, and to prevent the holding member 40 from kinking on the proximal operating section side. For instance, both of the proximal section 43 and the outer tube member 37 can be provided for the catheter 3. Alternatively, only either one of the proximal section 43 and the outer tube member 37 can be provided for the catheter 3. Besides, the arrangement of the proximal section 43 and the outer tube member 37 can be omitted in the case where the first elongated body 31 is sufficiently provided with pushability and kink resistance.

Incidentally, while a form in which a solid bar member is utilized as the holding member is shown in the drawings, a hollow tubular member can also be utilized as the holding member, as will be described later. Besides, the proximal section 43 can similarly be composed of a hollow tubular member.

The distal end 41 of the holding member 40 is located on the distal side (the left side in FIG. 20) of the first elongated body 31, relative to the led-out part of the connection cable 63 from the opening 67 of the protective member 65. If a starting point of reshaping into a curved shape or the like by means of the holding member 40 is formed in the led-out part of the connection cable 63 from the opening 67, the connection cable 63 may suffer a damage such as breaking of wire, and a breakage or a deterioration in function of the image sensor part 61 contained in the protective member 65. In order to prevent such troubles from occurring, in the catheter 3, the distal end 41 of the holding member 40 is disposed at the above-mentioned position.

The holding member 40 is preferably disposed at least at the position in the extending direction of the first elongated body 31 (the left-right direction in FIGS. 19 and 20) where the effective expansive section 32a of the balloon 32 is located. Such an arrangement ensures that the shape of the outer peripheral surface of the balloon 32 can be reshaped in conjunction with the first elongated body 31 (refer to FIGS. 22A and 22B). This ensures that the catheter 3 is applicable not only to a natural ostium having a rectilinear inner cavity shape but also to a natural ostium having a curved inner cavity shape. As a result, while using a single catheter 3, it is possible to widely cope with natural ostia which are located at different parts in a living body.

Now, a method of using the catheter 3 according to the present embodiment will be described below, referring to FIGS. 22.

Prior to introduction of the catheter 3 into a living body, reshaping of the catheter 3 is conducted. The reshaping can be easily conducted, for example, by the operator's manual operation. Specifically, a distal portion of the catheter 3 is reshaped into a desired shape according to the anatomical shape of the path to the sinusitis to be treated.

Referring to FIG. 22C, the catheter 3 is introduced into the external nose, in the state of being kept in an external shape imparted by the reshaping. Concurrently with the operation of introducing the catheter 3 into the external nose, suctioning by the suctioning unit is started. The suctioning prevents adhesion of body fluid or secretions onto the image obtaining plane of the imaging unit.

Referring to FIG. 22A, keeping the suctioning by use of the suction device P', a distal-side portion of the catheter 3 and the balloon 32 are introduced into the vicinity of the natural ostium.

Referring to FIG. 22B, after the balloon 32 provided on the catheter 3 is guided to a stenosed part S of the natural ostium, the balloon 32 is inflated. By the inflation of the balloon 32 it is possible to force open the stenosed part S formed at the natural ostium. Incidentally, the positioning (alignment) of the balloon 32 can be carried out in the same procedure as that described in the embodiments above.

It is also possible to clean the image obtaining plane and the inside of the paranasal sinus by supplying a liquid such as water through the suction lumen 71 before or after the inflation of the balloon 32. This ensures removal of body fluid or secretions adhered onto the image obtaining surface of the CCD camera 33, thereby allowing the CCD camera 33 to obtain a clearer image.

After the treatment, the catheter 3 is removed from the living body. After the catheter 3 is taken out of the living body, the external shape of the catheter 3 can be returned into the original shape before reshaping or can be deformed into a different external shape. In the cases of using the catheter 3 to other natural ostia to be treated of the same patient or in the cases of using the catheter 3 for other patients, the catheter 3 can be reshaped into an external shape according to the inner cavity shape of the natural ostium to be treated, each time of such treatment.

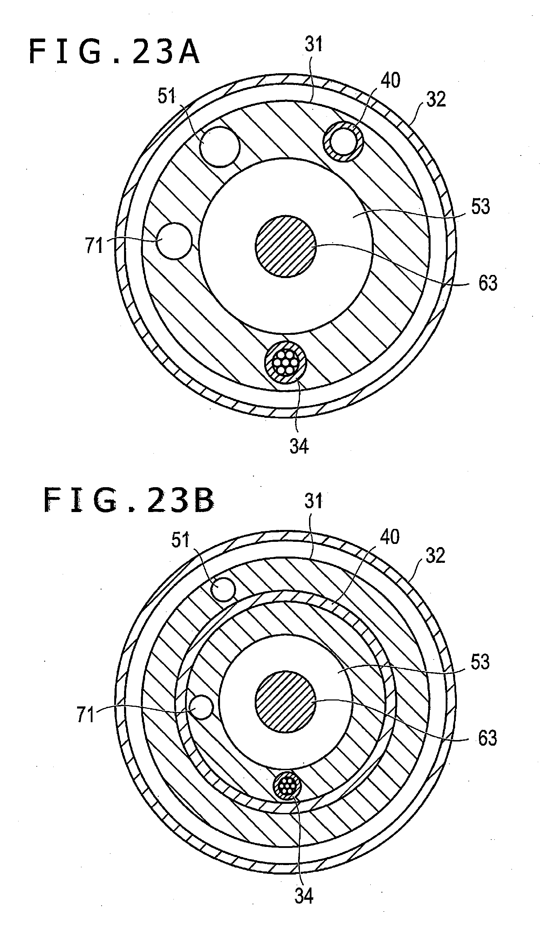

Now, the catheter according to Modification (1) of the catheter 3 in the present embodiment will be described below. FIGS. 23A and 23B are partial sectional views of the vicinity of the distal end of the catheter according to the present modification. FIGS. 23A and 23B correspond to sectional view along line VII-VII of FIG. 20.

As shown in FIG. 23A, a holding member 40 can be composed of a hollow tubular member, unlike that in the catheter 3 described above. Or, as shown in FIG. 23B, a holding member 40 can be disposed in a first elongated body 31 so as to cover the peripheries of a CCD camera lumen 53 and an optical fiber 34.

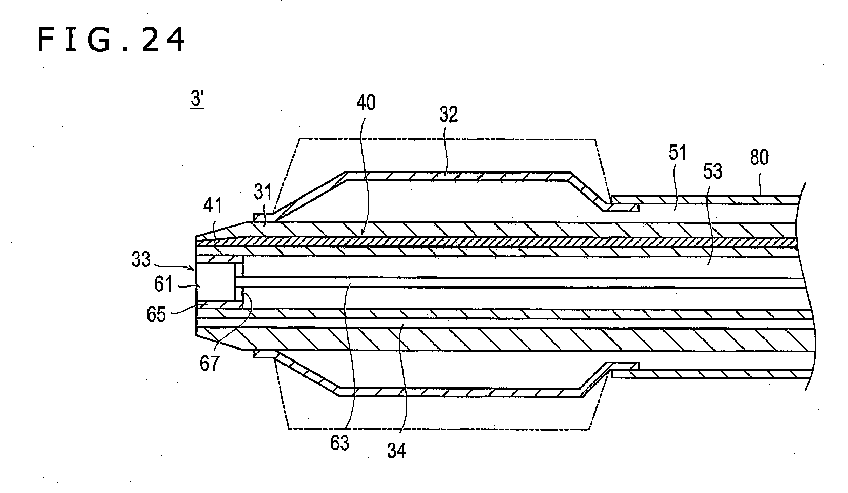

Now, a catheter 3' according to Modification (2) of the catheter 3 in the present embodiment will be described below. FIG. 24 is a longitudinal sectional view of the vicinity of the distal end of the catheter 3'.

In the catheter 3', a pressurizing medium lumen 51 is not formed inside a first elongated body 31 but is formed between the first elongated body 31 and a elongated body 80 which is disposed to cover the first elongated body 31 for the purpose of defining the pressurizing medium lumen 51. With the pressurizing medium lumen 51 not formed inside the first elongated body 31, it is possible for the first elongated body 31 to be made smaller in diametral size.

The elongated body 80 for defining the pressurizing medium lumen 51 is formed from the same or similar material to that of the first elongated body 31. In addition, a predetermined part of a balloon 32 is attached to the elongated body 80, with the inside of the balloon 32 kept in a liquid-tight and air-tight condition.

The distal end 41 of a holding member 40 is tapered distally. Where a protective member 65 is formed of a metal or the like, the sum of the rigidity possessed by the protective member 65 and the rigidity possessed by the holding member 40 is added to the distal end of the first elongated body 31. In such a case, the rigidity at the distal end of the first elongated body 31 may become excessively high, making it difficult to deform that portion into an arbitrary shape by manual operation. In view of this, by lowering the rigidity at the distal end of the holding member 40, the rigidity of the first elongated body 31 is made to be uniform along the longitudinal direction so that the first elongated body 31 can be deformed into an arbitrary shape on the distal end side. Incidentally, where the holding member 40 of the same material is utilized, the rigidity of the holding member 40 can be controlled by evenly decreasing or increasing the diameter of the holding member 40 along the longitudinal direction.

In addition, as shown in the figure, the first elongated body 31 may be tapered near the distal end in conformity with the tapered shape of the holding member 40. Such a configuration enables the catheter 3' to be smoothly introduced, advanced and retracted in a living body.

As shown in Modification (1) and Modification (2) above, the catheter according to the present embodiment can be appropriately modified, insofar as a long-shaped member having such a rigidity as to enable reshaping of the first elongated body 31 by manual operation or the like and to hold the first elongated body 31 in the reshaped state is used as the holding member. Other modifications which can be adopted to include a form wherein a plurality of comparatively short holding members are jointed in the longitudinal direction to be used as a single elongated body, and a form wherein the axis of the first elongated body 31 and the axis of the holding member 40 are not set in parallel and wherein the holding member 40 is embedded in the first elongated body 31 in the state of being curved in the circumferential direction of the first elongated body 31.

Besides, in each of the catheters in the third embodiment, also, the catheter may be provided with a marker, in the same manner as in the first embodiment and the second embodiment. An operation of positioning (aligning) the balloon by use of the marker can be incorporated into the treatment procedure by use of each of the catheters according to the third embodiment.

This application claims priority to Japanese Patent Application No. 2011-072998 filed on March 29, 2011, the entire contents of which are incorporated by reference herein.

### Description of Letters or Numerals

- 1: CATHETER (TREATMENT DEVICE)
- 11: FIRST ELONGATED BODY
- 11a: FIRST LUMEN
- 12: BALLOON (EXPANSION BODY)
- 12a: EFFECTIVE EXPANSIVE SECTION
- 12b1: TAPERED SECTION
- 12b2: TAPERED SECTION
- 13: CCD CAMERA (IMAGING UNIT)
- 13a: IMAGE OBTAINING PLANE
- 14: LED LIGHT (FIRST LIGHTING UNIT)
- 15: HUB (OPERATING SECTION)
- 151: BASE END PORTION
- 152: INDICATION SECTION
- 15a: IMAGE PORT
- 15b: PRESSURE SUPPLY PORT
- 15c: LIGHT SUPPLY PORT
- 16: SECOND ELONGATED BODY
- 16a: SECOND LUMEN
- 17: THIRD ELONGATED BODY
- 17a: THIRD LUMEN
- 18: FOURTH ELONGATED BODY
- 18a: FOURTH LUMEN
- 2: CATHETER (TREATMENT DEVICE)
- 21: FIRST ELONGATED BODY
- 22: BALLOON (EXPANSION BODY)
- 23: CCD CAMERA (IMAGING UNIT)
- 24: FIRST LED LIGHT (FIRST LIGHTING UNIT)
- 26: SECOND ELONGATED BODY
- 27: THIRD ELONGATED BODY
- 28: SECOND LED LIGHT (SECOND LIGHTING UNIT)
- 34: OPTICAL FIBER (FIRST LIGHTING UNIT)
- 40: HOLDING MEMBER
- 61: IMAGE SENSOR PART
- 63: CONNECTION CABLE
- 65: PROTECTIVE MEMBER
- 67: OPENING OF PROTECTIVE MEMBER
- 71: SUCTION LUMEN
- 80: ELONGATED BODY HAVING LUMEN FOR PRESSURIZING MEDIUM
- A: INSIDE OF PARANASAL SINUS
- D: DISPLAY DEVICE
- E: VINCINITY OF ENTRANCE
- I: FLUIDS SEND DEVICE
- L: LIGHT SOURCE DEVICE
- M: MARKER
- N: NASAL CAVITY
- O: NASAL CAVITY OPENING
- P: PRESSURE SUPPLY DEVICE
- P': SUCTION DEVICE
- S: STENOSED PART

## Claims

1. An otorhinolaryngological treatment device comprising:
a flexible elongated body to be inserted into a living body;
an expansion body which is provided on the elongated body and has an effective expansive section capable of radial expansive deformation within a natural ostium located between a nasal cavity and a paranasal sinus in the living body to force open a stenosed part of the natural ostium; and
imaging unit for obtaining an image on the front side of a distal end of the elongated body, which is provided integrally with the elongated body on the distal side of the elongated body relative to the expansion body.

2. The treatment device according to claim 1, comprising a long-shaped holding member contained in the elongated body and having a rigidity by which the external shape of the elongated body in the living body is held such that at least a part of the elongated body is bent into a direction crossing an axial direction of the elongated body.

3. The treatment device according to claim 2,
wherein the imaging unit includes an image sensor part disposed inside the elongated body, and a protective member which has an opening part for leading out a connection cable electrically connected to the image sensor part and which covers the image sensor part; and
a distal end of the protective member is disposed on the distal side of the elongated body relative to a part of the connection cable which is led out through the opening part.

4. The treatment device according to claim 2 or claim 3, wherein the holding member is disposed at least at a position in the extending direction of the elongated body where the effective expansive section of the expansion body is located.

5. The treatment device according to any one of claims 1 to 4, wherein the elongated body is provided with a marker for confirming that the elongated body is moved along the extending direction of the natural ostium by the distance between an image obtaining plane of the imaging unit and the effective expansive section.

6. The treatment device according to claim 5, wherein the marker is configured to be movable along the extending direction of the elongated body and to be fixable to the elongated body.

7. The treatment device according to claim 5 or claim 6, wherein the marker is configured to be detachable from the elongated body.

8. The treatment device according to any one of claims 5 to 7, wherein the marker has graduations for indicating the distance.

9. The treatment device according to any one of claims 1 to 8, wherein the elongated body is provided with a first lighting unit for illuminating the front side of the distal end of the elongated body.

10. The treatment device according to any one of claims 1 to 9, wherein the expansion body has a light-permeability; and
a second lighting unit for illuminating the outer periphery of the expansion body through the expansion body is disposed inside the expansion body.

11. The treatment device according to any one of claims 1 to 10, wherein a center position in the radial direction of the expansion body and a center position of an image obtaining plane of the imaging unit are coaxially-arranged with each other.

12. The treatment device according to any one of claims 1 to 11, wherein the imaging unit is located at the distal end of the elongated body.

13. A method of dilating a stenosed part of a natural ostium located between a nasal cavity and a paranasal sinus in a living body, comprising:
a step of introducing into the living body a elongated body provided with an expansion body capable of expansive deformation and contractive deformation, and an imaging unit for obtaining an image of the inside of the living body, disposed on the distal side of the elongated body relative to the expansion body, each of which is integrated with the elongated body;
a guiding step of obtaining an image on the front side of a distal end of the elongated body by the imaging unit, and guiding an effective expansive section of the expansion body for applying a pressure to the stenosed part of the natural ostium, into the stenosed part of the natural ostium on the basis of the obtained image; and
a dilating step of expanding the expansion body to thereby dilate the stenosed part of the natural ostium.

14. The method of dilating a stenosed part according to claim 13, wherein the guiding step includes a step of positioning an image obtaining plane of the imaging unit on the proximal side of an entrance to the paranasal sinus, and thereafter advancing the elongated body distally by the distance between the image obtaining plane and the effective expansive section of the expansion body thereby to position the effective expansive section with respect to the stenosed part of the natural ostium.

15. The method of dilating a stenosed part according to claim 13 or claim 14, wherein the dilating step includes at least a step of causing expansive deformation and contractive deformation in the expansion body while advancing the elongated body distally or retreating the elongated body proximally.

16. The method of dilating a stenosed part according to any one of claims 13 to 15, further comprising a suctioning step for suctioning a fluid in the living body through a lumen disposed in the elongated body in a state where the elongated body has been introduced into the living body.
